# NEW EUROPEAN PATENT SPECIFICATION

(11) **EP 0 591 647 B2**
(45) Date of publication and mention of the opposition decision: **21.04.2004**
(45) Mention of the grant of the patent: 05.07.2000
(21) Application number: 93112868.0
(22) Date of filing: 11.08.1993
(51) Int. Cl.: A61F 13/15

(54) **Stretchable absorbent article**
Dehnbarer absorbierender Gegenstand
Article absorbant extensible

(30) Priority: 13.08.1992 JP 23634292; 13.08.1992 JP 23634092
(43) Date of publication of application: 13.04.1994
(73) Proprietor: JAPAN ABSORBENT TECHNOLOGY INSTITUTE, Chuo-ku, Tokyo (JP)
(72) Inventor: Suzuki, Migaku, Kamakura-shi, Kanagawa (JP); Fukui, Hiroaki, Kawaguchi-shi, Saitama (JP)
(74) Representative: Gervasi, Gemma, Dr.

(56) References cited:
- EP-A- 0 119 825
- EP-A- 0 321 980
- EP-A- 0 321 985
- EP-A- 0 386 816
- EP-A- 0 449 271
- EP-A- 0 556 749
- GB-A- 2 249 712
- US-A- 4 781 966
- US-A- 4 883 549

## Description

### Technical Field

The present invention generally relates to absorbent articles such as diapers, sanitary products, and absorbent members suitable for use in such diapers, and more particularly to an elastically stretchable absorbent article.

### Background of the Invention

Disposable absorbent articles, such as diapers, sanitary products, incontinent pads, and the like have obtained wide acceptance by consumers. Such absorbent articles generally include a liquid permeable sheet, a liquid impermeable sheet for preventing body exudates from leaking out, and an absorbent member interposed therebetween for absorbing such body exudates.

Conventional liquid impermeable sheets provide satisfactory liquid impermeablility. However, those sheets are not flexible enough to closely conform to the shape of wearer's body, and consequently provide poor leakage protection.

Various types of materials have been proposed for use in absorbent members and may have been put into actual practice. Furthermore, with progress of development work on superabsorbent material comprising polymeric material capable of absorbing more than ten times its weight of liquids, absorbent members incorporating such super absorbent material have been widely utilized.

The absorbent members as have been already proposed or put into actual practice have a panel configuration for presenting a flat absorbent surface to be contacted by liquids over its entire area in an effort to obtain target liquid absorbency. Although such absorbent members provide satisfactory liquid absorbency, they are not flexible enough to conform to a wearer's body during their use. In particular, absorbent members incorporating superabsorbent material exhibit substantial increase in rigidity when the superabsorbent material absorbs liquids. This increase in rigidity of the absorbent members causes poor conformity thereof to the wearer's body, which leads to liquid leakage from the sides thereof.

From the closest prior art document EP321980 it is also known a stretchable absorbent article for filling about the waist of a wearer.

This article consists of a main body comprising an elastic composite sheet and an absorbent body secured thereto. The elastic composite sheet disclosed by EP321980 comprises an elastic sheet and a non-woven fabric secured to one surface of said elastic sheet. More specifically, the non-woven fabric is secured to one surface of the elastic sheet continuously in a first direction and intermittently in a second direction perpendicular to said first direction, in order to define therebetween a plurality of parallel channels extending in said first direction.

The present invention provides a highly-functional absorbent article which is at the same time elastically stretchable and contractible to better conform to the wearer's body during use.

### Summary of The Invention

The present invention provides a stretchable absorbent article as claimed in claim 1 and an absorbent article as claimed in claims 9 and 11.

The present invention further provides a stretchable absorbent member as claimed in daim 16.

The present invention according to claim 1 provides a stretchable absorbent artide comprising dual layers of an elastic composite sheet, each of said elastic composite sheets comprising one of said elastic sheet and one of said non woven fabrics. On the contrary, EP-A556749 (which is a document according to Art. 54(3) EPC) refers to a stretchable article providing a composite sheet which comprises a sheet backing material which is non-woven fabric impermeable layer, whilst the present stretchable absorbent article comprises an elastic composite sheet which comprises a non-woven fabric, which is a permeable layer.

Other features and advantages of the present invention will become readily apparent from the following detailed description, the accompanying drawings, and the appended claims.

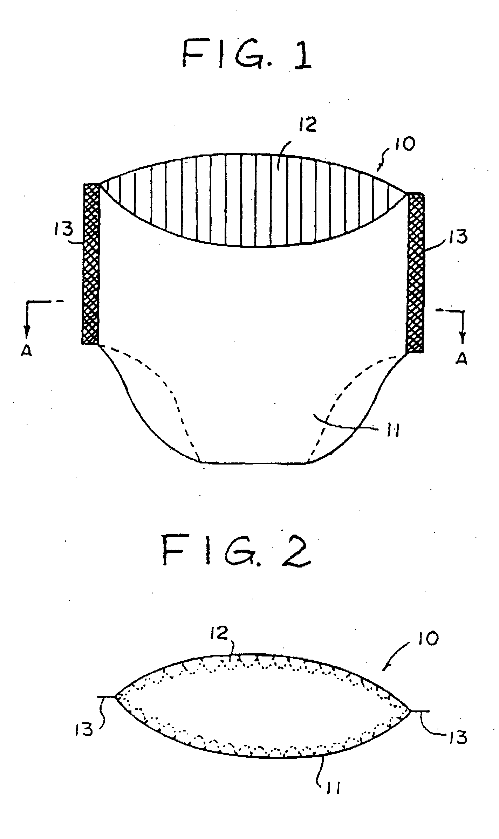

Referring to the features shown in the following Figures 1 to 4, 10, 13-15, 18, 20 and 21, as far as a composite sheet comprising only one elastic sheet and a non woven fabric is depicted, such features are not covered by claim 1. However, the figures are annexed for the sake of a better description of the claimed products.

### Brief Description of The Drawings

FIGURE 1 is a perspective view illustrating a first embodiment of liquid impermeable protective article.
FIGURE 2 is a cross-sectional view taken along a line A - A of FIGURE 1;
FIGURE 3 is a fragmentary perspective view of a composite sheet of the protective article of FIGURE 1;
FIGURE 4 is a perspective view of a second embodiment of a liquid impermeable protective article in accordance with the present invention;
FIGURE 5 is a cross-sectional view taken along a line B -B of FIGURE 4;
FIGURE 6 is a front view of a third embodiment of a liquid impermeable protective article in accordance with the present invention;
FIGURE 7 is a cross-sectional view taken along a line C - C of FIGURE 6;
FIGURE 8 is a front view of fourth embodiment of a liquid impermeable protective article of the present invention;
FIGURE 9 is a cross-sectional view taken along a line D - D of FIGURE 8;
FIGURE 10 is a cross-sectional view of a fifth embodiment of a liquid impermeable protective article.
FIGURE 11 is a cross-sectional view illustrating an absorbent body and attachment means thereof in the liquid impermeable protective article of FIGURE 10;
FIGURE 12 is a cross-sectional view illustrating another embodiment of a absorbent body and attachment means thereof in the liquid impermeable protective article of FIGURE 10;
FIGURE 13 is a cross-sectional view illustrating a sixth embodiment of a liquid impermeable protective article.
FIGURE 14 is fragmentary enlarged cross-sectional view of FIGURE 13;
FIGURE 15 is a perspective view illustrating a seventh embodiment of a liquid impermeable protective article
FIGURE 16 is a cross-sectional view illustrating a preferred construction of a securement portion of a liquid impermeable protective article in accordance with the present invention;
FIGURE 17 is a explanatory view illustrating various securement patterns applied to a securement portion of liquid impermeable protective article in accordance with the present invention;
FIGURE 18 is fragmentary perspective view illustrating one embodiment of the absorbent member.
FIGURE 19 is a fragmentary enlarged cross-sectional view of the absorbent member of FIGURE 18;
FIGURE 20 is a cross-sectional view illustrating another embodiment of the absorbent member.
FIGURE 21 is a cross-sectional view illustrating still another embodiment of the absorbent member.
FIGURE 22 is a cross-sectional view illustrating one mold embodiment which is employed to manufacture the absorbent member in accordance with the present invention;
FIGURE 23 is a cross-sectional view illustrating another mold embodiment which is employed to manufacture the absorbent member in accordance with the present invention.

### Detailed Description

While the present invention is susceptible of embodiment in various forms, there is shown in the drawings and will hereinafter be described in detail specific embodiments thereof, with the understanding that the present disclosure is to be considered as an exemplification of the invention, and is not intended to limit the invention to the specific embodiments illustrated.

Referring now to the drawings, FIGURE 1 is a perspective view illustrating an absorbent article, such as a liquid impermeable protective article of a pant type, embodying the principles of the present invention. FIGURE 2 is a cross-sectional view taken along the line A-A of FIGURE 1. In FIGURES 1 and 2, the liquid impermeable protective article is provided with a main body 10 which comprises a first portion 11 having a configuration covering a front face of a body waist and a second portion 12 for covering a back face of the body waist. These first and second portions 11, 12 are formed by cutting a composite sheet 1, as will be hereinafter described, into a generally-rectangular piece, bi-folding the piece along a longitudinal center portion and joining the folded portions to each other at joint portions 13 located at respective lateral ends of the folded portions.

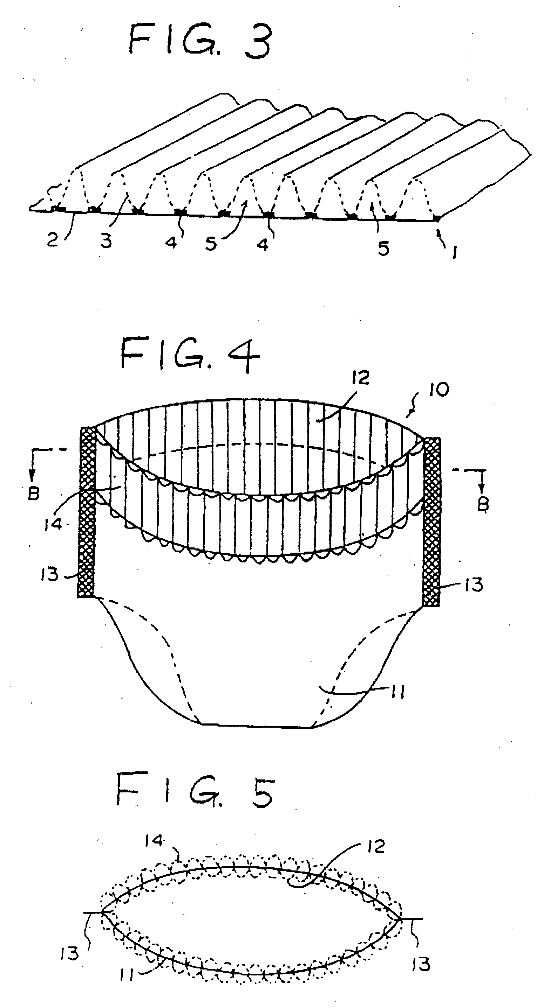

The composite sheet 1 constituting the first and second portions 11, 12 is constructed by securing a non-woven fabric 3 onto at least one surface of a liquid impermeable elastic sheet 2, as shown in FIGURE 3. The non-woven fabric 3 is secured to the elastic sheet 2 continuously in a first direction and intermittently in a second direction transverse to the first direction. The securement of these two elements are made along parallel securement lines 4 spaced from each other such as at regular intervals. A width of the non-woven fabric 3 is formed to be greater than that of the elastic sheet 2 between adjacent securement lines 4, so that a plurality of parallel channels 5 extending in the first direction are formed between the elastic sheet 2 and the non-woven fabric 3. This composite sheet 1 is cut or contoured in accordance with desired shapes and dimensions of the first and second portions 11, 12 which are then joined to each other so as to form the non-woven fabric 3 to face inwardly.

In a liquid impermeable protective article 10 of such construction, the inwardly placed non-woven fabric 3 contacts the body of a wearer when the article 10 is applied to the wearer and a plurality of elongated channels 5 provided between such non-woven fabric 3 and the elastic sheet 2. Accordingly, breathability and flexibility of the non-woven fabric 3 per se and the channels 5 provide a comfortable feeling during use, by softness to skin. The outwardly placed elastic sheet 2 is liquid impermeable, so that liquids are effectively prevented from leaking through the article.

Although the main body 10 comprising the first and second portions 11, 12 is illustrated as being single-layered in its entire region in the embodiment of FIGURE 1, it may be dual-layered (i.e., doubled) by placing a belt-like strengthening member 14 of a suitable width cut from the composite sheet 1 onto respective upper end portions of the first and second portions 11, 12, as illustrated in FIGUREs 4 and 5. This construction increases strength as well as elastic contractibility in such portions, so that any breakage which may be caused by a careless handling of the article during use will be avoided. Its stability about the body waist during application will also be improved. The securement of the main body 10 and the strengthening member 14 may be readily made by the use of heat compression, adhesives of the hotmelt type and the like. Such strengthening may alternatively be accomplished by forming the main body 10 longer than a desired dimension and folding its upper end portion onto itself.

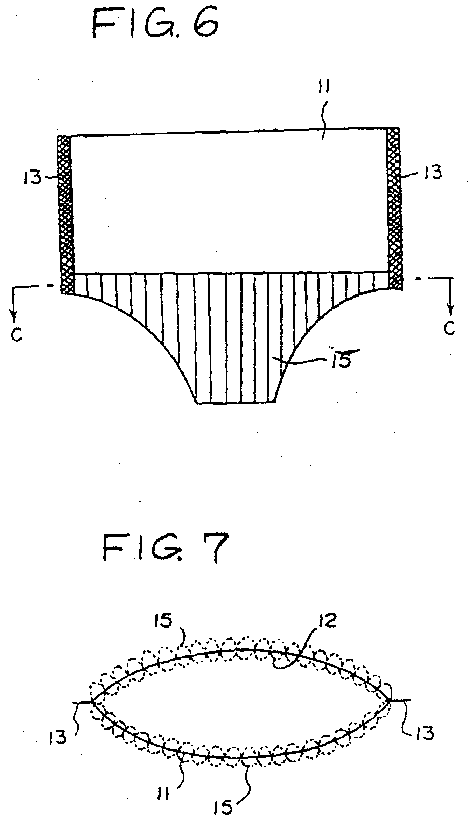

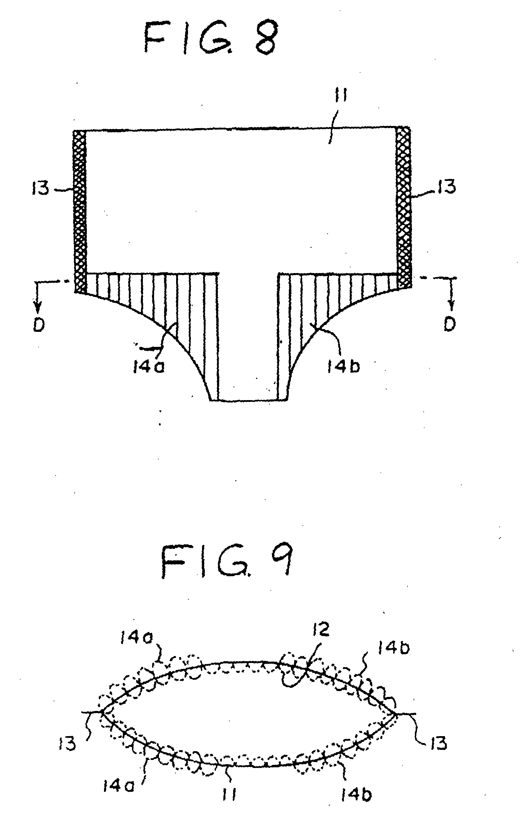

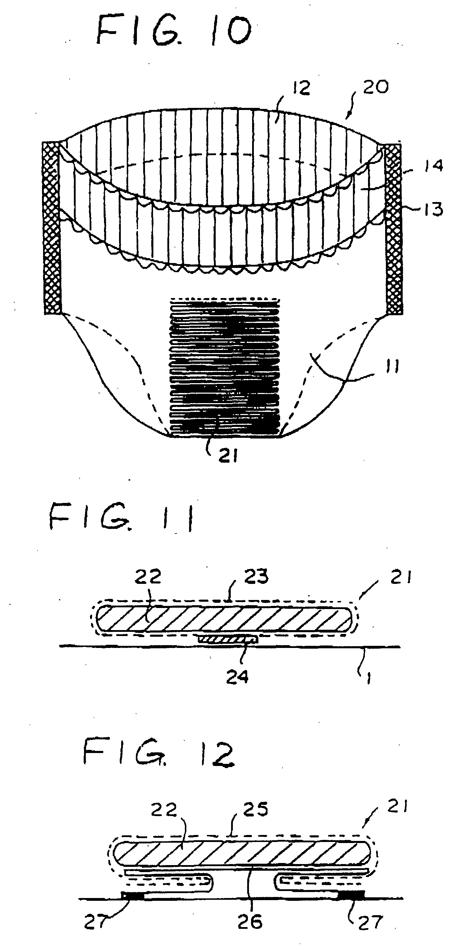

Alternatively, the main body 10 may be dual-layered in its crotch region by providing a strengthening member 15 having a shape corresponding to such region, as illustrated in FIGURE 6 and 7. In still another embodiment, reinforcing members 14a, 14b may be respectively attached to two portions surrounding the leg holes, as illustrated in FIGUREs 8 and 9. In either embodiment, the leg hole portions, to which a relatively great stretching force is applied when the article is put on or removed from the wearer, are constructed to be dual-layered, so that breakage in such portions is effectively prevented. The crotch or leg hole strengthening portion may be combined with a waist strengthening portion as illustrated in FIGURE 4.

FIGURE 10 shows another embodiment of a liquid impermeable protective article in accordance with the present invention. The liquid impermeable protective article 20 of this embodiment has the main body 10 of the construction, in which the first portion 11 and the second portion 12 are joined to each other at joint portions 13, formed with the folded portion 14 in its upper end portion, similar to the construction shown in FIGURE 4. The article 20 is also provided with an absorbent body 21 positioned in its crotch region. The absorbent body 21 may be of the type as employed for conventional diapers or sanitary napkins and fixedly secured in a predetermined position by means such as glues, hotmelt adhesives and the like.

Such an absorbent body as described above is per se rigid, and accordingly lacks elasticity and flexibility like the composite sheet 1 has, with the result that elasticity in a portion of the main body 10 is disturbed when such a rigid absorbent body is attached to the portion of the main body of the present article. The attachment as illustrated in FIGURE 11 and 12 may be preferably utilized in order to avoid such drawbacks.

The absorbent body 21, as illustrated in FIGURE 11, comprises an absorbent member 22 and a liquid permeable cover 23 for enclosing the absorbent member 22. The absorbent body 21 is at its transverse center portion secured to the non-woven fabric side of the composite sheet 1 only along a band-like elongated securement portion 24 extending a full length of the absorbent body, and formed such as by adhesives, hotmelts and the like. Such construction permits the composite sheet to be less affected by the securement of the absorbent body 21 thereto and allows the absorbent body 21 to be freed from the stretching and contracting action of the composite sheet. Accordingly, the construction permits any deformation of the absorbent body along a wearer's body shape and accommodates a dimensional change of the absorbent member after liquid absorption. Furthermore, the absorbent body does not move or deviate from the predetermined position. Therefore, leakage is completely prevented and a very comfortable feeling is achieved.

Alternatively, the absorbent body 21 may comprise a liquid permeable topsheet 25 for covering top and side surfaces of the absorbent member 22 and a backsheet 26 for covering a bottom surface of the absorbent member 22, as illustrated in FIGURE 12. Portions of the topsheet 25 and the backsheet 26 extending from each side edge of the absorbent member 22 are folded beneath the absorbent member 22 to take a U-shaped configuration. The portions are secured to the composite sheet 1 at band-like securement portion 27 which are located at leading ends of the extension portions, and are formed such as by adhesives, hotmelts and the like. In this construction, the absorbent member 22 takes a position as if it "floats" above the composite sheet so that movement of the absorbent member 22 substantially independent of movement of the composite sheet. This provides about the same or higher degrees of leakage protection and comfort than the construction of FIGURE 10.

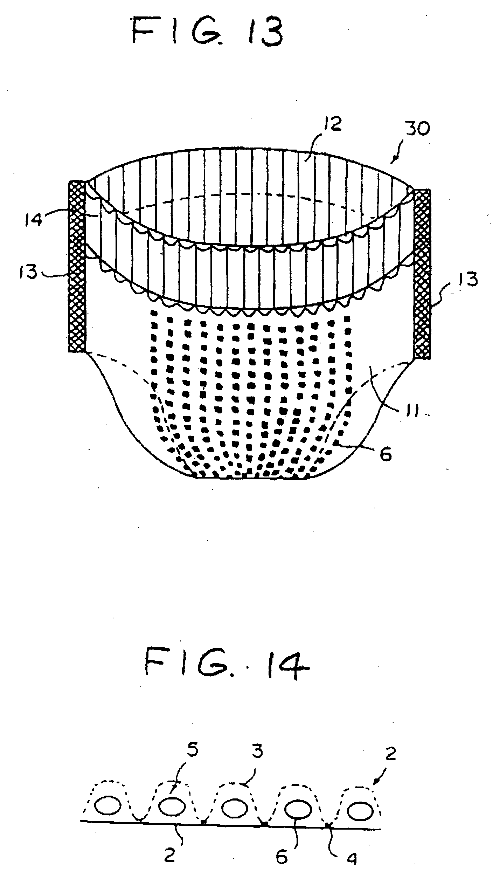

FIGURE 13 shows another embodiment of the liquid impermeable protective article 30 which is intended to further enhance the effects of the present invention. In this embodiment, the composite sheet 1 includes absorbent material 6 positioned within channels defined between the elastic sheet 2 and the non-woven fabric 3, as illustrated in FIGURE 14. The absorbent material 6 may be positioned in the entire region of the composite sheet 1. Alternatively, the absorbent material 6 may be positioned only in a crotch region of the composite sheet 1 with which contact with body exudate is possible, as illustrated in FIGURE 13. Such absorbent material 6 absorbs body exudates which have passed through the non-woven fabric 3 and entered into channels 5. This eliminates the need to provide a separate absorbent body.

Suitable absorbent material 6 for placement within the channels 5 includes hydrophilic material such as wood pulp or cotton, or synthetic fibrous material such as polypropylene, polyester and the like which is hydrophilized by hydrophilic treatment. Preferably, such absorbent material utilizes superabsorbent material which is generally referred to as S.A.P. (Super Absorbent Polymer) capable of absorbing several times its own weight of water or aqueous liquids.

In the event the superabsorbent material is utilized in a particulate form, the non-woven fabric constituting walls of the channels 5 is required to be of high quality in terms of a filtering function to prevent the particulates from passing therethrough. Preferably, the superabsorbent material may be incorporated in the absorbent material in various forms. One example would be strips of suitable widths cut from a composite sheet material comprising superabsorbent material particles and wood pulp or a fibrous web. Another example is a bundle of fibrous superabsorbent material, such as RUNSEAL manufactured and marketed by Toyobo Corp. under such trade name. Strips slit from a fibrous web with superabsorbent material deposited on its surface may also be employed. Such material may be manufactured by polymerizing and cross-linking a monomer solution placed on the fibrous web surface.

The volume of the absorbent material increases as it absorbs liquids. In particular, the superabsorbent material exhibits a drastic volume change before and after liquid absorption. Therefore, when a cross-sectional area of the absorbent material is selected to substantially correspond to that of the channel space, the channel would be enlarged by the amount that the superabsorbent material swells as it absorbs liquids. Such enlargement would be readily accommodated primarily by a stretching action of the elastic sheet. However, it is preferable that such a cross-sectional area of the absorbent material is selected within a range such that the cross-sectional area after swelling remains smaller than that of the channel space when the swelling of the absorbent material is drastic, such as with superabsorbent material, or such volume change is not desirable. Such range selection creates a free space within the channel, so that superabsorbent material is able to swell freely without restriction and fully utilize its absorbent capacity.

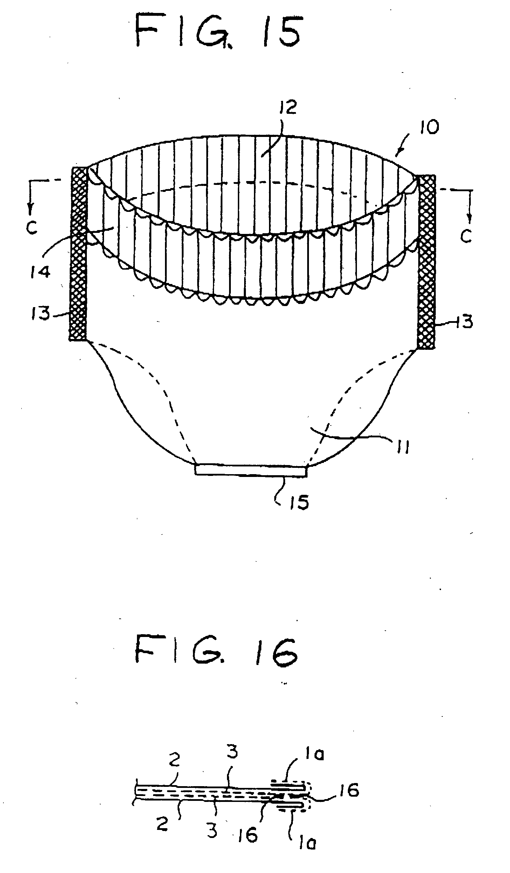

In respective embodiments as illustrated in FIGUREs 1 - 14, the main body is constructed by forming leg holes on a generally-rectangular single composite sheet 1, bi-folding the sheet 1 along its longitudinal center portion and joining or securing the folded portions to each other at their respective opposite side ends. In an alternative embodiment as shown in FIGURE 15, the main body 10 is constructed by securing the first and second portions 11, 12 separately cut from the composite sheet along side end securement portions 13 and a lower end securement portion 15.

In respective embodiments as described above, the securement portions for securing the first and second portions to each other may be formed by securing opposing inwardly-facing non-woven fabrics 3 to each other by suitable securement means such as heat-sealing, adhesives of the hotmelt type and the like. Preferably, the main body 10 is, at portions corresponding to the securement portions, folded outwardly to define folded portions 1 a having a suitable width, preferably equal to that of the securement portion. The folded portions 1a are secured together along securement lines 16. In this construction, the U-folded non-woven fabrics 3 are positioned at side edges of respective securement portions so that those portions provide soft and smooth surfaces such as against an outside clothing thereby providing a more comfortable feeling during wearing as contrasted to the previous construction wherein cut edges of the elastic sheet 2 project to those portions.

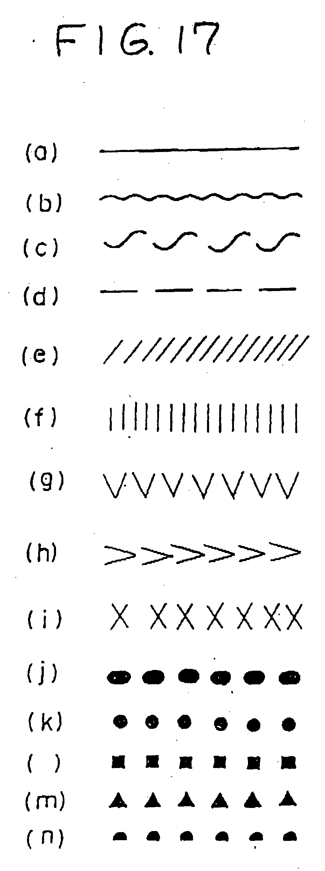

In addition, the securement of the composite sheet 1 to itself at securement portions 13, 15 may be made along two securement lines 16 parallel to each other, as illustrated in FIGURE 16. Alternatively, the securement may be made in such patterns (a) - (n) as illustrated in FIGURE 17. Particularly, intermittent patterns such as (c) - (n) in FIGURE 17 provide lower strength than continuous patterns. Such intermittent patterns may be preferably utilized for articles such as diaper covers which are desired to be removed from a wearer by breaking the main body 10.

It has been found that the very flexible nature of the composite sheet allows folding thereof into a compact form so that carrying and use thereof are facilitated.

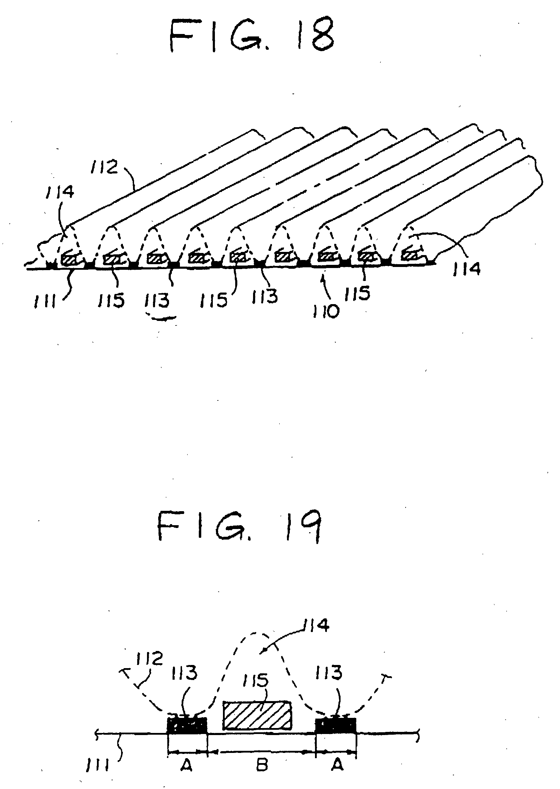

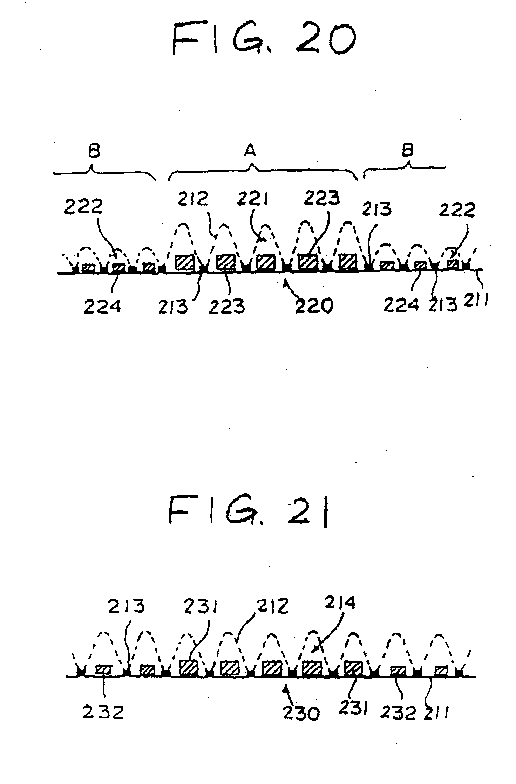

FIGURE 18 is a perspective view illustrating an absorbent member embodying the principles of the present invention. FIGURE 19 is a fragmentary enlarged cross-sectional view of FIGURE 10. In FIGURE 18 and 19, an absorbent member 110 in a sheet form comprises an elastic sheet 111 and a non-woven fabric 112 which are secured together continuously in a first direction of the non-woven fabric 112, such as in a machine direction, and intermittently in a second direction perpendicular to the first direction, i.e.in a cross-machine direction, along securement lines 113. A width of the non-woven fabric 112 between neighboring securement lines 113 is selected to be greater than that of the elastic sheet 111 so that a plurality of channels 114 extending in the machine direction are formed between the elastic sheet 111 and the non-woven fabric 112.

An absorbent material 115, such as of an elongated band-like configuration cut from superabsorbent sheet is positioned in the channels 114 so as to extend a full length of the respective channel 114. A cross-sectional area of the absorbent material 115 is selected to be about 1/2 or smaller, for example 1/5 of that of the channel 114.

The absorbent member 110 thus constructed is placed in actual use so that the non-woven fabric 112 contacts liquids. For example, when the absorbent member 110 is incorporated in a diaper or sanitary napkin, the non-woven fabric 112 serves as a topsheet for facing the body of the wearer and the elastic sheet 111 serves as a liquid impermeable backsheet for preventing liquid leakage. The liquid is absorbed by the absorbent materials 115 positioned in the channels 114.

FIGURE 20 illustrates another embodiment of the absorbent member in accordance with the present invention. The absorbent member 220 in this embodiment has two different channels 221, 222 of different cross-sectional areas. A cross-sectional area of each of the first channels 221, which are positioned in a laterally central portion A of the absorbent sheet 220, is greater than that of the second channels 222 which are positioned in lateral side portions B on opposite sides of the laterally central portion A. A cross-sectional area of first absorbent materials 223 placed in the first channels 221 is proportionally greater than that of second absorbent materials 224 placed in the second channels 222.

The absorbent member 220 of the construction as illustrated in FIGURE 20, accordingly has a higher liquid absorbency in the central portion A than in the side portions B. This feature is especially suitable for use in articles having a narrow central region to which liquids are discharged in a concentrated manner.

The channel structure of the absorbent members as illustrated in FIGURES 18 - 21 is in its dry state (before liquid absorption) effective in enhancing diffusion and flow-in of liquids and air in a longitudinal direction, and in its wet state (after liquid absorption) effective in enhancing liquid diffusion to rapidly diffuse liquids received therein in the longitudinal direction, with the result that liquids are distributed within entire regions of the absorbent members placed in the channels in a short period of time, and effective absorbency of the absorbent sheet is exhibited.

Means for securing the elastic sheet 211 and the non-woven fabric 212 so as to form channels 214 are not limited to any specific manner. For example, the pre-shaped wavy non-woven fabric 212 may be placed on the flat elastic sheet 211 so as to contact to each other. The contact portions are then secured together by any suitable means such as adhesives, sonic-sealing, heat-compression and the like. The most preferable method is to finish securement by placing the non-woven fabric in contact with the elastic sheet while it is tacky in its manufacturing process.

Alternative means for forming channels may utilize a difference between machine-directional and cross machine-directional elongation rates of the non-woven fabric. Specifically, the non-woven fabric is placed on at least one surface of the elastic sheet. They are secured together continuously in the machine direction and intermittently in the cross-machine direction to form the composite sheet. The composite sheet is then stretched in a cross-machine direction at a ratio exceeding an elastic limit of the non-woven fabric so that a permanent elongation is created in the non-woven fabric. When released, the channels are formed in the composite sheet since the permanently elongated non-woven fabric has a width greater than a width of the elastic sheet between the neighboring securement lines. Non-woven fabrics having a higher elongation rate in the cross-machine direction than in the machine direction such as illustrated as No.3 and No.4 in Table I are suitably used for such method for forming channels.

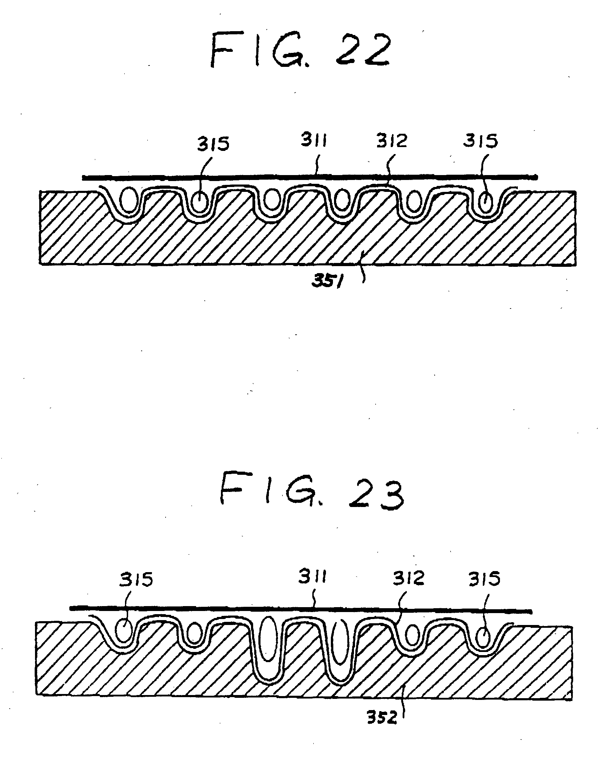

The placement of the absorbent material in channel structures may be performed by positioning the absorbent material between the elastic sheet and the non-woven fabric before they are secured together to form channels. However, in order to place the absorbent material in precise positions in actual industrial practice, a method as illustrated in FIGURES 22 and 23 may be preferably employed. The non-woven fabric 312 is tucked into a mold 351 or 352 having rows of grooves, mechanically or by vacuum suction so that a plurality of spaced top surface portions and channels between the adjacent top surface portions are provided. A plurality of individual absorbent materials 315 are then placed in the respective channels of the non-woven sheet 312. Finally, the elastic sheet 311 is secured onto the spaced top surface portions of the non-woven fabric 312 to enclose the individual absorbent materials 315 within the respective channels. The mold 351 illustrated in FIGURE 22 has a plurality of grooves of equal cross-sectional area into which the individual absorbent materials of equal cross-sectional area are positioned. On the other hand, the mold 352 illustrated in FIGURE 23 has central grooves having a greater cross-sectional area than that of side grooves on opposite sides of the central grooves. Likewise, the individual absorbent materials 315 positioned in the central grooves have a greater cross-sectional area than that in the side grooves.

Additionally, the composite absorbent member in accordance with the present invention has a construction such that absorbent materials of high liquid absorbency are positioned in the channels defined between the elastic sheet and the non-woven fabric. Liquids pass through the non-woven fabric into the channels where they are absorbed by the absorbent materials.

When it is desired that a selected portion of the absorbent surface of the absorbent member of the present invention absorb higher volume of liquids than the remaining portions of the absorbent surface, the amount of the absorbent materials positioned in the channels located in the selected portion may be increased. Such density distribution of the absorbent members may preferably be made by varying cross-sectional areas of the channels for enclosing absorbent materials in selected portions of the absorbent surface in accordance with a desired distribution. The absorbent member of such construction is particularly suitable for use in infant diapers and sanitary napkins which have a central target zone into which liquids are heavily discharged.

The elastic sheet for use in the absorbent member of the present invention includes thin-layered sheet such as films consisting of a material selected from a natural or synthetic rubber, polyurethane, styrene-butadiene block polymer, EVA, LLDPE of ultra low density ethylene-propylene elastomer, ethylene-methylacrylate elastomer and a mixture thereof with a styrene-ethylene-butadiene-styrene block copolymer; co-extruded films of polyurethane elastomers and polyolefine elastomers; and polyurethane meltblown non-woven fabrics.

The non-woven fabric for use in the absorbent member of the present invention is selected by considering firstly its function as an absorbent and secondly its ability to form channels.

In order for the non-woven fabric to function as an absorbent, it needs to be liquid permeable without adverse result during use against skin. Its adhesive characteristic with film, and its ability to maintain a channel configuration, should be taken into consideration from a view point of the ability to form channels.

Based on the above consideration, the following non-woven fabrics may be advantageously employed; non-woven fabrics of a lower basis weight (preferably lower than 50 g/m²) manufactured by methods such as dry-laid, spun-bond, spunlace and the like, from fibers of relatively low denier (preferably lower than 5d) comprising hydrophobic synthetic fibers such as polypropylene, polyester, nylon and the like, hydrophilic fibers such as rayon, cotton and the like, or any combination thereof.

Non-woven fabric predominantly comprising hydrophobic fibers may be preferably treated by surfactants which are safe to skin to render the fabrics highly liquid permeable. When the non-woven fabric predominantly comprises hydrophilic fibers such as rayon fibers, it is desirable that such hydrophilic fibers comprise anti-bacterial fibers that are safe to the skin for the purpose of preventing adverse effects to skin which may be caused by high water content in a surface layer of the hydrophilic fibers. Such commercially available fibers as CHITOCELL® or CHITOPOLY® manufactured by Fuji Spinning Corp. may be effectively used.

Table I provides a list of typical non-woven fabrics for use in the absorbent member of the present invention. Particularly, the spunlaced non-woven fabrics such as listed as No.3 in Table I are particularly suited for the intended purpose since they exhibit a high elongation rate in a width direction of the channel and also have a high strength.

Exemplified non-woven fabrics suitable for the present invention are illustrated in Table I.

**TABLE 1**

| No. | Type of N.W. | | Raw fiber | | | Web | |
|---|---|---|---|---|---|---|---|
| 1 | spunbonded | | PPx2.2dx continuous filament | | | random | |
| 2 | thermally-bonded | | PPx2.2dx35m/m | | | random | |
| 3 | spunlaced | | PPx1.5dx45m/m | | | parallel | |
| 4 | tow | | acetate tow | | | parallel | |

| No.Basis weight | | | Strength(kg/2.5cm) | | | Elongation(%) | |
|---|---|---|---|---|---|---|---|
| | (g/m²) | MD | CD | MD/CD | MD | CD | MD/CD |
| 1 | 30 | 5.3 | 2.2 | 2.4 | 30 | 48 | 1.6 |
| 2 | 32 | 3.9 | 0.6 | 6.5 | 21 | 70 | 3.3 |
| 3 | 35 | 3.7 | 0.8 | 4.6 | 20 | 230 | 11.6 |
| 4 | 40 | 5.2 | 0.4 | 13 | 15 | 290 | 19.3 |

As described above, the absorbent member of the present invention is constructed by securing the non-woven fabric to at least one face of the highly-stretchable, liquid impermeable elastic sheet continuously in one direction and intermittently in a direction perpendicular to the one direction along the securement portions, forming a plurality of channels extending parallel to each other between the non-woven fabric and the elastic sheet, and positioning the absorbent members in the channels. Accordingly, the absorbent member has excellent elastic and water-proof characteristics. Also the non-woven fabric of the absorbent member provides excellent flexibility and breathability characteristics. Therefore, the present absorbent member is most suitable for use in a variety of articles which contact skin during use, such as disposable diapers, sanitary napkins and the like which are applied to the waist or crotch of the wearer.

It is to be understood that no limitation with respect to the specific embodiment illustrated herein is intended or should be inferred. The disclosure is intended to cover by the appended claims all such modifications as fall within the scope of the claims.

## Claims

1. A stretchable absorbent article for fitting about the waist of a wearer, said article comprising:
a main body (10) consisting of an elastic composite sheet (1) and an absorbent body secured thereto, said elastic composite sheet (1) comprising an elastic sheet (2) and a non-woven fabric (3) secured to one surface of said elastic sheet (2),
said non-woven fabric layer (3) being secured to said elastic sheet (2) continuously in a first direction and intermittently in a second direction perpendicular to said first direction to define therebetween a plurality of parallel channels (5) extending in said first direction, said stretchable absorbent article, including dual layers of said elastic composite sheet (1) in the region of at least one of a front waist portion (11), a rear waist portion (12), a crotch portion, and portions surroundings leg holes of said main body (10), each of said elastic composite sheets (1) comprising one of said elastic sheets (2) and one of said non-woven fabrics (3).

2. A stretchable absorbent article of claim 1, **characterized in that** said absorbent body is mounted inside said main body (10).

3. A stretchable absorbent article of claim 1, wherein
said elastic sheet (2) is a thin-layered sheet selected from a film consisting of a material selected from a natural or synthetic rubber, polyurethane, styrene-butadiene block polymer, EVA, LLDPE of ultra low density ethylene-propylene elastomer, ethylene-methylacrylate elastomer and a mixture thereof with a styrene-ethylene-butadiene styrene block copolymer; a co-extruded film of polyurethane elastomers and polyolefine elastomers; and a polyurethane meltblown non-woven fabric.

4. A stretchable absorbent artide of claim 1, wherein
said non-woven fabric (3) comprises hydrophilic synthetic fibers selected from a group consisting of rayon and cotton.

5. A stretchable absorbent article of claim 1, wherein
said non-woven fabric (3) comprises hydrophobic synthetic fibers selected from a group consisting of polypropylene, polyester, and nylon.

6. A stretchable absorbent article of claim 1, wherein
said non-woven fabric (3) comprising hydrophobic synthetic fibers is treated with surfactants.

7. A stretchable absorbent article of daim 3, wherein
said absorbent body is arranged to longitudinally extend interior of a crotch portion of the absorbent article, and is secured to an inner surface of a crotch portion at a band-like elongated securement portion (24) thereof positioned centrally transversely thereof.

8. A stretchable absorbent artide of claim 3, wherein
said absorbent body is arranged to longitudinally extend interior of a crotch portion of the absorbent article, and further extends outwardly of respective transverse sides thereof, said absorbent body being secured to an inner surface of the crotch portion at free ends of the backsheet.

9. An absorbent article comprising a stretchable region, including a liquid permeable layer (4) , an elastic layer (6) stretchably secured to said liquid permeable layer to form regions of non-securement between adjacent ones of said regions of securement, said liquid permeable layer forming corrugations at the respective regions of non-securement for defining respective channels (114) between said liquid permeable layer and said elastic layer, **characterised in that** said elastic layer is a liquid impermeable layer (111), that the absorbent article comprises stretchable regions including the said permeable layer and said impermeable elastic layer, that said liquid permeable layer and said liquid impermeable layer are secured to one another at discrete regions, and that absorbent material (115) is disposed in selected ones of said channels.

10. An absorbent article according to claim 9, **characterized in that** said liquid permeable layer is a top sheet (3), said liquid impermeable elastic layer is a backsheet (2) having an elastic area and said discrete regions of securement consist of a plurality of spaced, parallel lines of securement (4) by which said elastic area of the backsheet (2) is secured to said topsheet (3), said absorbent material being disposed in said channels in a selected location of said absorbent article.

11. An absorbent article comprising a liquid permeable top sheet (3) ; an elastic layer being stretchably secured to said liquid permeable topsheet by a plurality of spaced, parallel lines of securement (4) so as to form regions of non securement, said liquid permeable layer respectively forming corrugations at said regions of non-securement for defining a plurality of channels (5) between said liquid permeable topsheet and said elastic layer, **characterised in that** the elastic layer constitutes the liquid impermeable backsheet (2) of the absorbent article, that the impermeable backsheet has an elastic area being secured to said topsheet, and that the absorbent material is disposed in said channels in a selected location of said absorbent article.

12. An absorbent article of claim 11, wherein said selected location is a crotch region of the absorbent article.

13. An absorbent article of claim 12, wherein said selected location is a lateral central region of the absorbent article.

14. An absorbent article of claim 13, wherein said parallel lines of securement (4) are laterally spaced from each other to define said channels (5) longitudinally extending between front and rear regions of said absorbent article.

15. An absorbent article of claim 14, wherein said absorbent material (6) comprises a plurality of absorbent segments longitudinally arranged in said channel.

16. A stretchable absorbent member comprising a stretchable region including a liquid permeable layer (212), an elastic layer (211) stretchably secured to said liquid permeable layer to form regions of non-securement between adjacent ones of said regions of securement, said liquid permeable layer forming corrugations at the respective regions of non-securement for defining respective channels (214) between said liquid permeable layer and said elastic layer, **characterised In that** said elastic layer is a liquid impermeable layer, that the absorbent member comprises stretchable regions including the said permeable layer and said impermeable elastic layer, that said liquid permeable layer and said liquid impermeable layer are secured to one another at discrete regions, and that absorbent material (232) is disposed in selected ones of said channels.

17. A stretchable absorbent member of claim 16, wherein said absorbent material comprises superabsorbent material.

18. A stretchable absorbent member of claim 17, wherein
said liquid permeable layer comprises a non-woven fabric.

19. A stretchable absorbent member according to claim 16, **characterized in that** said discrete regions of securement consist of a plurality of spaced, parallel lines of securement.

20. A stretchable absorbent member of claim 19 , wherein
said absorbent material has a preshaped configuration having a substantially uniform lateral crass-sectional area.

21. A stretchable absorbent member of claim 20, wherein
said preshaped absorbent material comprises hydrophilic fibers end superabsorbent polymers.

22. A stretchable absorbent member of claim 21, wherein
said preshaped absorbent material comprises a bundle of superabsorbent fibers.

23. A stretchable absorbent member of claim 21, wherein
said lateral cross-sectional area of the preshaped absorbent material is equal to or less than a half of a lateral cross-sectional area of the channel encasing said absorbent material.

24. A stretchable absorbent member of claim 21, wherein
said absorbent member has a plurality of lateral portions, and said channels in one of said lateral portions have a greater lateral cross-sectional area than the channels in an adjacent lateral portion.

25. A stretchable absorbent member of claim 24, wherein
said channels in a central lateral portion of the absorbent structure have a greater lateral cross-sectional area than the channels in lateral portions on opposite sides of said central lateral portion.

26. A stretchable absorbent member of claim 25, wherein
said absorbent material enclosed in the channels in one of said lateral portions have a greater lateral cross-sectional area than the absorbent material in the channels in the adjacent lateral portion.

## Patentansprüche

1. Dehnbarer absorbierender Gegenstand zum Anbringen um die Taille eines Trägers herum, wobei der Gegenstand umfasst:
einen Hauptkörper (10), der aus einer elastischen Verbundlage (1) und einem absorbierenden Körper besteht, der daran befestigt ist,
wobei die elastische Verbundlage (1) eine elastische Lage (2) und einen nicht-gewebten Stoff (3) umfasst, der an einer Oberfläche der elastischen Lage (2) befestigt ist,
die nicht-gewebte Stoffschicht (3) an der elastischen Lage (2) kontinuierlich in einer ersten Richtung und intermittierend in einer zweiten Richtung rechtwinklig zu der ersten Richtung befestigt ist, um zwischeneinander eine Vielzahl von parallelen Kanälen (5) zu bilden, die sich in der ersten Richtung erstrecken, wobei
der dehnbare absorbierende Gegenstand Doppelschichten aus der elastischen Verbundlage (1) in dem Bereich mindestens eines Bereiches von vorderem Taillenbereich (11), von hinterem Taillenbereich (12), von Zwickelbereich und Bereichen, die Beinlöcher des Hauptkörpers (10) umgeben, aufweist, wobei jede der elastischen Lagen (1) eine der elastischen Lagen (2) und einen der nicht-gewebten Stoffe (3) umfasst.

2. Dehnbarer absorbierender Gegenstand nach Anspruch 1, **dadurch gekennzeichnet, daß** der absorbierende Körper innenseitig des Hauptkörpers (10) angebracht ist.

3. Dehnbarer absorbierender Gegenstand nach Anspruch 1, wobei
die elastische Lage (2) eine dünn geschichtete Lage ist ausgewählt aus einer Folie, die aus einem Material besteht ausgewählt aus natürlichem oder synthetischem Gummi, Polyurethan, Styrol-Butadien-Blockpolymer, EVA, LLDPE aus Ethylen-Propylen-Elastomer ultrageringer Dichte, Ethylen-Methylacrylat-Elastomer und einer Mischung hieraus mit einem Styrol-Ethylen-Butadien-Styrol-Blockpolymer; einer coextrudierten Folie aus Polyurethan-Elastomeren und Polyolefin-Elastomeren; und einem nicht-gewebten Stoff aus schmelzgeblasenem Polyurethan.

4. Dehnbarer absorbierender Gegenstand nach Anspruch 1, wobei
der nicht-gewebte Stoff (3) hydrophile synthetische Fasern ausgewählt aus einer Gruppe bestehend aus Rayon und Baumwolle umfasst.

5. Dehnbarer absorbierender Gegenstand nach Anspruch 1, wobei
der nicht-gewebte Stoff (3) hydrophobe synthetische Fasem ausgewählt aus einer Gruppe bestehend aus Polypropylen, Polyester und Nylon umfasst.

6. Dehnbarer absorbierender Gegenstand nach Anspruch 1, wobei
der nicht gewebte Stoff (3), der hydrophobe synthetische Fasern umfasst, mit oberflächenaktiven Mitteln behandelt ist.

7. Dehnbarer absorbierender Gegenstand nach Anspruch 3, wobei
der absorbierende Körper in Hinblick darauf angeordnet ist, dass er sich in Längsrichtung innenseitig eines Zwickelbereichs des absorbierenden Gegenstandes erstreckt, und an der inneren Oberfläche des Zwickelbereichs an einem bandartigen länglichen Befestigungsbereich (24) desselben befestigt ist, der zentral querliegend hierzu angeordnet ist.

8. Dehnbarer absorbierender Gegenstand nach Anspruch 3, wobei
der absorbierende Körper in Hinblick darauf angeordnet ist, dass er sich in Längsrichtung innenseitig eines Zwickelbereichs des absorbierenden Gegenstandes erstreckt und sich femer außenseitig der jeweiligen querliegenden Seiten desselben erstreckt, wobei der absorbierende Körper an der inneren Oberfläche des Zwickelbereichs an den freien Enden der rückseitigen Lage befestigt ist.

9. Absorbierender Gegenstand, umfassend einen dehnbaren Bereich, der eine für eine Flüssigkeit permeable Schicht (4), eine elastische Schicht (6), die dehnbar an der für eine Flüssigkeit permeablen Schicht zur Bildung von Bereichen ohne Befestigung zwischen benachbarten Bereichen von Befestigungsbereichen befestigt ist, wobei die für eine Flüssigkeit permeable Schicht Wellen an den jeweiligen Bereichen ohne Befestigung zur Ausbildung jeweiliger Kanäle (114) zwischen der für eine Flüssigkeit permeablen Schicht und der elastischen Schicht bildet, **dadurch gekennzeichnet, dass** die elastische Schicht eine für eine Flüssigkeit impermeable Schicht (111) ist, dass der absorbierende Gegenstand dehnbare Bereiche umfasst, die die permeable Schicht und die impermeable elastische Schicht aufweisen, dass die für eine Flüssigkeit permeable Schicht und die für eine Flüssigkeit impermeable Schicht an diskreten Bereichen miteinander befestigt sind und dass das absorbierende Material (115) in ausgewählten Kanälen der Kanäle angeordnet ist.

10. Absorbierender Gegenstand nach Anspruch 9, **dadurch gekennzeichnet, daß** die für eine Flüssigkeit permeable Schicht eine obere Lage (3) ist, dass die für eine Flüssigkeit impermeable elastische Schicht eine rückseitige Lage (2) mit einem elastischen Bereich ist und diskrete Bereiche zur Befestigung aus einer Vielzahl von beabstandeten, parallelen Befestigungslinien (4) bestehen, mittels der der elastische Bereich an der rückseitigen Lage (2) der oberen Lage (3) befestigt ist, wobei absorbierendes Material in den Kanälen an einer ausgewählten Stelle des absorbierenden Gegenstandes angeordnet ist.

11. Absorbierender Gegenstand, umfassend eine für eine Flüssigkeit permeable obere Lage (3); eine elastische Schicht, die an der für eine Flüssigkeit permeablen oberen Lage mittels einer Vielzahl von beabstandeten, parallelen Befestigungslinien (4) in Hinblick darauf dehnbar befestigt ist, Bereiche ohne Befestigung zu bilden, wobei die für eine Flüssigkeit permeable Schicht jeweils Wellen an den Bereichen ohne Befestigung zur'Ausbildung einer Vielzahl von Kanälen (5) zwischen der für eine Flüssigkeit permeable oberen Lage und der elastischen Schicht bildet, **dadurch gekennzeichnet, dass** die elastische Schicht die für eine Flüssigkeit impermeable rückseitige Lage (2) des absorbierenden Gegenstandes bildet, dass die impermeable rückseitige Lage einen elastischen Bereich aufweist, die an der oberen Lage befestigt ist, und dass das absorbierende Material in den Kanälen an einer ausgewählten Stelle des absorbierenden Gegenstandes angeordnet ist.

12. Absorbierender Gegenstand nach Anspruch 11, wobei die ausgewählte Stelle ein Zwickelbereich des absorbierenden Gegenstandes ist.

13. Absorbierender Gegenstand nach Anspruch 12, wobei die ausgewählte Stelle ein querliegender zentraler Bereich des absorbierenden Gegenstandes ist.

14. Absorbierender Gegenstand nach Anspruch 10, wobei die parallelen Befestigungslinien (4) querliegend voneinander zur Bildung der Kanäle (5) beabstandet sind, die sich in Längsrichtung zwischen dem vorderen und dem hinteren Bereich des absorbierenden Gegenstandes erstrecken.

15. Absorbierender Gegenstand nach Anspruch 14, wobei das absorbierende Material (6) eine Vielzahl von absorbierenden Segmenten umfasst, die in Längsrichtung in dem Kanal angeordnet sind.

16. Dehnbares absorbierendes Teil, umfassend eine für eine Flüssigkeit permeable Schicht (212), eine elastische Schicht (211), die an der für eine Flüssigkeit permeablen Schicht befestigt ist, um Bereiche ohne Befestigung zwischen benachbarten Bereichen der Befestigungsbereiche zu bilden, wobei die für eine Flüssigkeit permeable Schicht Wellen an jeweiligen Bereichen ohne Befestigung zur Ausbildung zugehöriger Kanäle (214) zwischen der für eine Flüssigkeit permeablen Schicht und der elastischen Schicht bildet, **dadurch gekennzeichnet, daß** die elastische Schicht eine für eine Flüssigkeit impermeable Schicht ist, daß das absorbierende Element dehnbare Bereiche umfasst, die die permeable Schicht und die impermeable elastische Schicht aufweisen, daß die für eine Flüssigkeit permeable Schicht und die für eine Flüssigkeit impermeable Schicht an diskreten Bereichen miteinander befestigt sind und dass absorbierendes Material (232) in ausgewählten Kanälen der Kanäle angeordnet ist.

17. Dehnbares absorbierendes Teil nach Anspruch 16, wobei das absorbierende Material superabsorbierendes Material umfasst.

18. Dehnbares absorbierendes Teil nach Anspruch 17, wobei die für eine Flüssigkeit permeable Schicht einen nicht-gewebten Stoff umfasst.

19. Dehnbares absorbierendes Teil nach Anspruch 16, **dadurch gekennzeichnet, dass** die diskreten Befestigungsbereiche aus einer Vielzahl von beabstandeten, parallelen Befestigungslinien bestehen.

20. Dehnbares absorbierendes Teil nach Anspruch 19, wobei
das absorbierende Material eine vorgeformte Konfiguration aufweist, die eine im Wesentlichen gleichmäßige, querliegende Querschnittsfläche besitzt.

21. Dehnbares absorbierendes Teil nach Anspruch 20, wobei
das vorgeformte absorbierende Material hydrophile Fasern und superabsorbierende Polymere umfasst.

22. Dehnbares absorbierendes Teil nach Anspruch 21, wobei
das vorgeformte absorbierende Material ein Bündel von superabsorbierenden Fasern umfasst.

23. Dehnbares absorbierendes Teil nach Anspruch 21, wobei
die querliegende Querschnittsfläche des vorgeformten absorbierenden Materials gleich oder kleiner als die Hälfte der querliegenden Querschnittsfläche des Kanals ist, der das absorbierende Material umschließt.

24. Dehnbares absorbierendes Teil nach Anspruch 21, wobei
das absorbierende Teil eine Vielzahl von seitlichen Bereichen aufweist und die Kanäle in einem Bereich der querliegenden Bereiche eine größere querliegende Querschnittsfläche als die Kanäle in einem benachbarten querliegenden Bereich aufweisen.

25. Dehnbares absorbierendes Teil nach Anspruch 24, wobei
die Kanäle in einem querliegenden zentralen Bereich der absorbierenden Struktur eine größere querliegende Querschnittsfläche als die Kanäle in den querliegenden Bereichen an gegenüberliegenden Seiten des querliegenden zentralen Bereichs aufweisen.

26. Dehnbares absorbierendes Teil nach Anspruch 25, wobei
das absorbierende Material, das in den Kanälen in einem Bereich der querliegenden Bereiche enthalten ist, eine größere querliegende Querschnittsfläche als das absorbierende Material in den Kanälen in dem benachbarten querliegenden Bereich aufweist.

## Revendications

1. Article absorbant extensible destiné à s'ajuster autour de la taille de la personne qui le porte, ledit article comprenant :
un corps principal (10) consistant en une feuille composite élastique (1) et un corps absorbant fixé sur celle-ci,
ladite feuille composite élastique (1) comprenant une feuille élastique (2) et un tissu non tissé (3) fixé sur une surface de ladite feuille élastique (2),
ladite couche de tissu non tissé (3) étant fixée sur ladite feuille élastique (2) en continu dans une première direction et en discontinu dans une seconde direction perpendiculaire à ladite première direction de manière à définir entre elles plusieurs canaux parallèles (5) s'étendant dans ladite première direction, ledit article absorbant extensible incluant des couches doubles de ladite feuille composite élastique (1) dans au moins une région parmi la partie avant de la taille (11), la partie arrière de la taille (12), la partie entrejambe et les parties entourant les orifices pour les jambes dudit corps principal (10), chacune desdites feuilles composites élastiques (1) comprenant une desdites feuilles élastiques (2) et un desdits tissus non tissés (3).

2. Article absorbant extensible selon la revendication 1, **caractérisé en ce que** ledit corps absorbant est monté à l'intérieur dudit corps principal (10).

3. Article absorbant extensible selon la revendication 1, dans lequel ladite feuille élastique (2) est une feuille en couche mince choisie parmi un film constitué par une matière choisie parmi le caoutchouc naturel ou synthétique, le polyuréthanne, un polymère bloc styrène - butadiène, l'EVA, un LLDPE d'élastomère éthylène - propylène d'ultra faible densité, un élastomère éthylène - acrylate de méthyle et un de leurs mélanges avec un copolymère bloc styrène - éthylène - butadiène - styrène ; un film co-extrudé d'élastomères de polyuréthanne et d'élastomères polyoléfiniques ; et un tissu non tissé à base de polyuréthanne obtenu par fusion soufflage.

4. Article absorbant extensible selon la revendication 1, dans lequel ledit tissu non tissé (3) comprend des fibres synthétiques hydrophiles choisies dans le groupe constitué par la rayonne et le coton.

5. Article absorbant extensible selon la revendication 1, dans lequel ledit tissu non tissé (3) comprend des fibres synthétiques hydrophobes choisies dans le groupe constitué par le polypropylène, le polyester et le Nylon.

6. Article absorbant extensible selon la revendication 1, dans lequel ledit tissu non tissé (3) comprenant des fibres synthétiques hydrophobes est traité par des tensioactifs.

7. Article absorbant extensible selon la revendication 3, dans lequel ledit corps absorbant est disposé de façon à s'étendre longitudinalement à l'intérieur de la partie entrejambe de l'article absorbant, et est fixé sur la surface intérieure de la partie entrejambe à une partie de fixation allongée du type bande (24) de celle-ci positionnée en son centre et transversalement.

8. Article absorbant extensible selon la revendication 3, dans lequel ledit corps absorbant est disposé de façon à s'étendre longitudinalement à l'intérieur de la partie entrejambe de l'article absorbant, et s'étend en outre vers l'extérieur des côtés transversaux respectifs de celle-ci, ledit corps absorbant étant fixé sur la surface intérieure de la partie entrejambe aux extrémités libres de la feuille de support.

9. Article absorbant comprenant une région extensible, incluant une feuille perméable aux liquides (4), une couche élastique (6) fixée de façon extensible à ladite couche perméable aux liquides pour former des régions de non fixation entre des régions adjacentes desdites régions de fixation, ladite couche perméable aux liquides formant des ondulations aux régions respectives de non fixation pour définir des canaux respectifs (114) entre ladite couche perméable aux liquides et ladite couche élastique, **caractérisé en ce que** ladite couche élastique est une couche imperméable aux liquides (111), que l'article absorbant comprend des régions extensibles incluant ladite couche perméable et ladite couche élastique imperméable, que ladite couche perméable aux liquides et ladite couche imperméable aux liquides sont fixées l'une à l'autre en des régions discrètes et que la matière absorbante (115) est disposée dans certains desdits canaux choisis.

10. Article absorbant selon la revendication 9, **caractérisé en ce que** ladite couche perméable aux liquides est une feuille de couverture (3), ladite couche élastique imperméable aux liquides est une feuille de support (2) ayant une zone élastique et lesdites régions discrètes de fixation consistent en plusieurs lignes parallèles, espacées, de fixation (4) grâce auxquelles ladite zone élastique de la feuille de support (2) est fixée à ladite feuille de couverture (3), ladite matière absorbante étant disposée dans lesdits canaux dans une zone choisie dudit article absorbant.

11. Article absorbant comprenant une feuille de couverture perméable aux liquides (3); une couche élastique extensible fixée à ladite feuille de couverture perméable aux liquides par plusieurs lignes parallèles, espacées, de fixation (4) de façon à former des régions de non fixation, ladite couche perméable aux liquides formant respectivement des ondulations aux dites régions de non fixation pour définir plusieurs canaux (5) entre ladite feuille de couverture perméable aux liquides et ladite couche élastique, **caractérisé en ce que** la couche élastique constitue la feuille de support imperméable aux liquides (2) de l'article absorbant, que la feuille de support imperméable a une zone élastique fixée à ladite feuille de couverture et que la matière absorbante est disposée dans lesdits canaux dans une zone choisie dudit article absorbant.

12. Article absorbant selon la revendication 11, dans lequel ladite zone choisie est la région de l'entrejambe de l'article absorbant.

13. Article absorbant selon la revendication 12, dans lequel ladite zone choisie est une région centrale latérale de l'article absorbant.

14. Article absorbant selon la revendication 13, dans lequel lesdites lignes parallèles de fixation (4) sont espacées latéralement les unes des autres pour définir lesdits canaux (5) s'étendant longitudinalement entre les régions avant et arrière dudit article absorbant.

15. Article absorbant selon la revendication 14, dans lequel ladite matière absorbante (6) comprend plusieurs segments absorbants disposés longitudinalement dans ledit canal.

16. Elément absorbant extensible comprenant une région extensible incluant une couche perméable aux liquides (212), une couche élastique (211) fixée de façon extensible à ladite couche perméable aux liquides pour former des régions de non fixation entre des régions adjacentes parmi les dites régions de fixation, ladite couche perméable aux liquides formant des ondulations aux régions respectives de non fixation pour définir des canaux respectifs (214) entre ladite couche perméable aux liquides et ladite couche élastique, **caractérisé en ce que** ladite couche élastique est une couche imperméable aux liquides, que l'élément absorbant comprend des régions extensibles incluant ladite couche perméable et ladite couche imperméable élastique, que ladite couche perméable aux liquides et ladite couche imperméable aux liquides sont fixées l'une à l'autre en des régions discrètes et que de la matière absorbante (232) est disposée dans certains desdits canaux choisis.

17. Elément absorbant extensible selon la revendication 16, dans lequel ladite matière absorbante comprend de la matière super absorbante.

18. Elément absorbant extensible selon la revendication 17, dans lequel ladite couche perméable aux liquides comprend un tissu non tissé.

19. Elément absorbant extensible selon la revendication 16, **caractérisé en ce que** lesdites régions discrètes de fixation consistent en plusieurs lignes de fixation parallèles, espacées.

20. Elément absorbant extensible selon la revendication 19, dans lequel ladite matière absorbante a une configuration pré-façonnée dont la surface de la section droite latérale est sensiblement uniforme.

21. Elément absorbant extensible selon la revendication 20, dans lequel ladite matière absorbante pré-façonnée comprend des fibres hydrophiles et des polymères super absorbants.

22. Elément absorbant extensible selon la revendication 21, dans lequel ladite matière absorbante pré-façonnée comprend un faisceau de fibres super absorbantes.

23. Elément absorbant extensible selon la revendication 21, dans lequel ladite surface de la section droite latérale de la matière absorbante pré-façonnée est égale ou inférieure à la moitié de la surface de la section droite latérale du canal dans lequel est enfermée ladite matière absorbante.

24. Elément absorbant extensible selon la revendication 21, dans lequel ledit élément absorbant a plusieurs parties latérales et lesdits canaux dans une desdites parties latérales ont une surface de section droite latérale supérieure aux canaux de la partie latérale adjacente.

25. Elément absorbant extensible selon la revendication 24, dans lequel lesdits canaux de la partie centrale latérale de la structure absorbante ont une surface de section droite latérale supérieure aux canaux des parties latérales de chaque côté de ladite partie centrale latérale.

26. Elément absorbant extensible selon la revendication 25, dans lequel ladite matière absorbante enfermée dans les canaux dans une desdites parties latérales a une surface de section droite latérale supérieure à celle de la matière absorbante dans les canaux de la partie latérale adjacente.
